(19) Europäisches Patentamt
European Patent Office
Office européen des brevets

(11) **EP 4 745 558 A1**

(12) **EUROPEAN PATENT APPLICATION**
published in accordance with Art. 153(4) EPC

(43) Date of publication:
**20.05.2026 Bulletin 2026/21**

(21) Application number: **23949790.2**

(22) Date of filing: **24.08.2023**

(51) International Patent Classification (IPC):
***G01N 21/27*** *(2006.01)*

(52) Cooperative Patent Classification (CPC):
**G01N 21/05; G01N 21/31; G01N 21/61;
G01N 33/0039;** G01N 2021/052; G01N 2201/062;
G01N 2201/08

(86) International application number:
**PCT/JP2023/030506**

(87) International publication number:
**WO 2025/041334 (27.02.2025 Gazette 2025/09)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **MITSUBISHI ELECTRIC
CORPORATION**
**Chiyoda-ku**
**Tokyo 100-8310 (JP)**

(72) Inventor: **OINUMA Gaku**
**Tokyo 100-8310 (JP)**

(74) Representative: **Parker, Andrew James**
**Meissner Bolte Partnerschaft mbB**
**Patentanwälte Rechtsanwälte**
**Postfach 86 06 24**
**81633 München (DE)**

(54) **OZONE CONCENTRATION MEASUREMENT DEVICE, OZONE CONCENTRATION MEASUREMENT METHOD, AND OZONE GENERATION SYSTEM**

(57) An ozone concentration measurement device (100) for measuring an ozone concentration of measurement target gas in a container (8) includes: a photoelectric sensor (4) in which a light emission section (2) for emitting light, a light reception section (3) for receiving the light, and a signal processing section (1) for transmitting a light emission signal to the light emission section (2) and receiving a light reception signal from the light reception section (3), are integrally formed; a light-emission optical fiber (5) which is connected to the light emission section (2) and introduces the light into the container; a light-reception optical fiber (6) which leads out the light having passed through inside of the container (8), to the light reception section (3); and a calculation section (9) which calculates the ozone concentration of the measurement target gas on the basis of the light reception signal from the signal processing section (1).

FIG. 1

## Description

### TECHNICAL FIELD

The present invention relates to an ozone concentration measurement device, an ozone concentration measurement method, and an ozone generation system.

### BACKGROUND ART

Ozone is used in a variety of fields such as water treatment, deodorization, semiconductor manufacturing, sterilization, and virus inactivation. In industrial usage of ozone, it is necessary to measure the concentration of ozone to be supplied, and an ozone concentration measurement device that is simple and has high accuracy is required. For example, as a method for measuring a high ozone concentration of 200 g/Nm3 or higher for ozone generated by an ozone generator, there is an ozone concentration measurement device in which ozone in a light-shielded container is irradiated with light from a visible light source, and the absorbance of ozone is measured by a light receiving element placed opposite to the visible light source, whereby the ozone concentration is measured (see, for example, Patent Document 1).

### CITATION LIST

### PATENT DOCUMENT

Patent Document 1:     Japanese Laid-Open Patent Publication JP 2012 -132 827 A

### SUMMARY OF THE INVENTION

### PROBLEM TO BE SOLVED BY THE INVENTION

In a conventional ozone concentration measurement device, a pair of light-transmitting windows is provided to a light-shielded container with an optical path length set at several cm to several tens of cm, and visible light including a wavelength of 550 nm to 630 nm is emitted from a visible light source provided on the outer side of one light-transmitting window toward the other light-transmitting window, and the visible light is detected by a light receiving element attached on the outer side of the other light-transmitting window with a light receiving surface of the light receiving element facing the visible light source, whereby the absorbance of ozone is measured.
Thus, the visible light source and the light receiving element are placed at locations away from each other by at least the optical path length. Accordingly, due to a temperature difference between both components, slight noise induced on electrical wiring connected to both components, and the like, the measured absorbance becomes unstable, resulting in low measurement accuracy for the ozone concentration. In addition, the visible light source and the light receiving element are provided on the outer sides of the pair of light-transmitting windows and power supplies for driving these are needed, so that the device is complicated and is increased in size.
The present invention has been made to solve the above problems, and an object of the present invention is to provide an ozone concentration measurement device, an ozone concentration measurement method, and an ozone generation system using these, which can measure an ozone concentration with high accuracy, using a small-sized and simple configuration.

### MEANS TO SOLVE THE PROBLEM

An ozone concentration measurement device according to the present invention is an ozone concentration measurement device for measuring a concentration of ozone of measurement target gas in a container, the ozone concentration measurement device including: a photoelectric sensor in which a light emission section for emitting light, a light reception section for receiving the light, and a signal processing section for transmitting a light emission signal to the light emission section and receiving a light reception signal from the light reception section, are integrally formed; a light-emission optical fiber which is connected to the light emission section and introduces the light into the container; a light-reception optical fiber which leads out the light having passed through inside of the container, to the light reception section; and a calculation section which calculates the concentration of ozone of the measurement target gas on the basis of the light reception signal from the signal processing section.

An ozone concentration measurement method according to the present invention is an ozone concentration measurement method for measuring a concentration of ozone of measurement target gas in a container, the ozone concentration measurement method including: a photoelectric sensor preparation step of preparing a photoelectric sensor in which a light emission section for emitting light, a light reception section for receiving the light, and a signal processing section for transmitting a light emission signal to the light emission section and receiving a light reception signal from the light reception section, are integrally formed; a light-emission optical fiber preparation step of preparing a light-emission optical fiber which is connected to the light emission section and introduces the light into the container; a light-reception optical fiber preparation step of preparing a light-reception optical fiber which leads out the light having passed through inside of the container, to the light reception section; and a calculation step of calculating a concentration of ozone of the measurement target gas on the basis of the light reception signal from the signal processing section.

An ozone generation system according to the present invention is an ozone generation system including: an ozone generator which generates and feeds ozone as the measurement target gas to outside; the above ozone concentration measurement device which introduces the measurement target gas fed from the ozone generator, into the container, and measures the concentration of ozone of the measurement target gas; and a control section which controls the ozone generator on the basis of a result of measurement for the concentration of ozone of the measurement target gas by the ozone concentration measurement device.

**EFFECT OF THE INVENTION**

With the ozone concentration measurement device, the ozone concentration measurement method, and the ozone generation system according to the present invention, it is possible to perform measurement of an ozone concentration with high accuracy, using a small-sized and simple configuration.

BRIEF DESCRIPTION OF THE DRAWINGS

FIG. 1 shows a configuration of an ozone concentration measurement device according to Embodiment 1.
FIG. 2 is a sectional view showing a configuration of a container part of the ozone concentration measurement device shown in FIG. 1.
FIG. 3 is a sectional view showing a configuration of a container part of an ozone concentration measurement device according to Embodiment 2.
FIG. 4 is a sectional view showing another configuration of a container part of the ozone concentration measurement device according to Embodiment 2.
FIG. 5 shows a light emission pattern in a light emission section of a photoelectric sensor of an ozone concentration measurement device according to Embodiment 3.
FIG. 6 shows a configuration of an ozone concentration measurement device according to Embodiment 4.
FIG. 7 shows a configuration of an ozone concentration measurement device according to Embodiment 5.
FIG. 8 shows a configuration of an ozone generation system according to Embodiment 6.
FIG. 9 shows a configuration of an ozone generation system according to Embodiment 7.
FIG. 10 is a block diagram showing another example of a photoelectric sensor according to each embodiment.
FIG. 11 is a block diagram showing an example of a configuration of hardware of a control section and a calculation section according to each embodiment.

**DESCRIPTION OF EMBODIMENTS**

Hereinafter, preferred embodiments of an ozone concentration measurement device, an ozone concentration measurement method, and an ozone generation system according to the present invention will be described with reference to the drawings. The same or corresponding matters and parts are denoted by the same reference characters, and the detailed description thereof is omitted as appropriate. In all the embodiments, similarly, components and operations denoted by the same reference characters will not be repeatedly described.

Embodiment 1

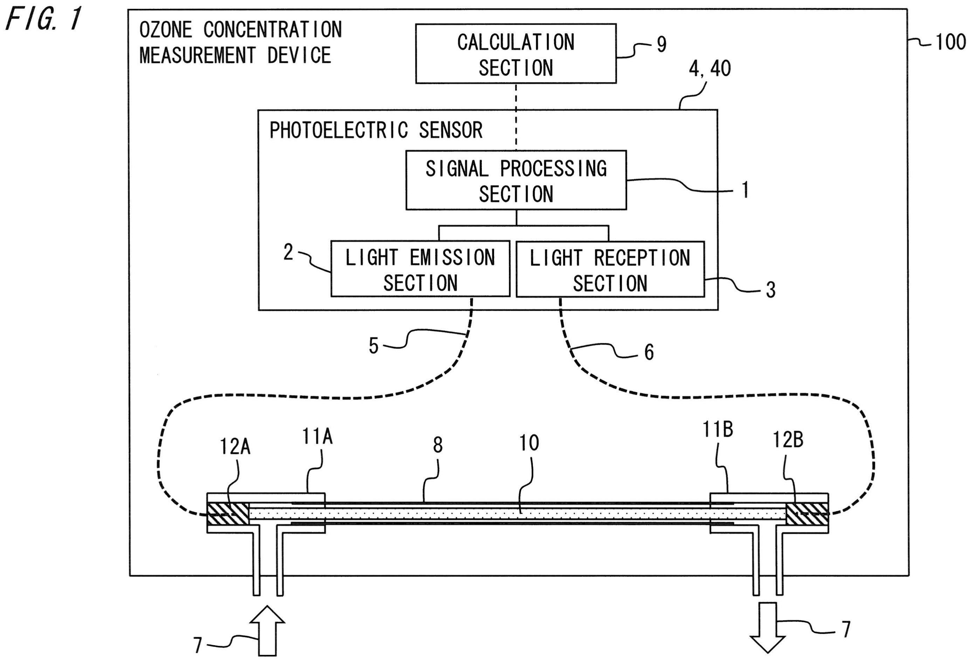

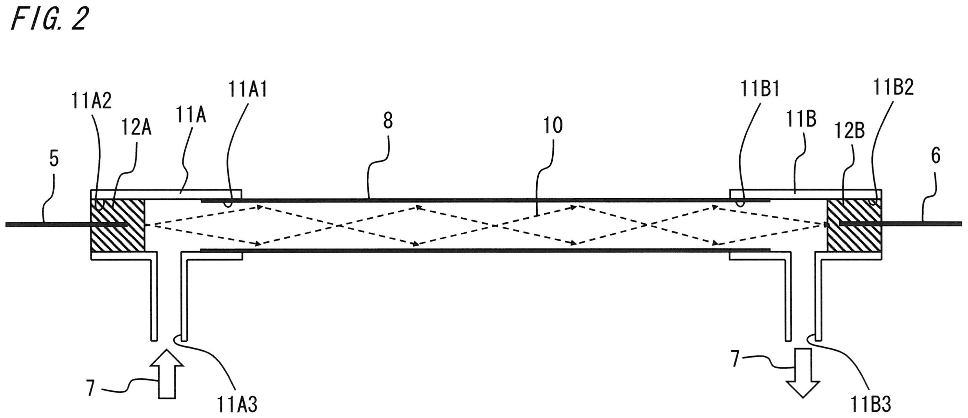

FIG. 1 shows a configuration of an ozone concentration measurement device according to Embodiment 1. FIG. 2 is a sectional view showing a configuration of a container part of the ozone concentration measurement device shown in FIG. 1.

As shown in FIG. 1, an ozone concentration measurement device 100 includes a photoelectric sensor 4, a light-emission optical fiber 5, a light-reception optical fiber 6, a container (also called a cell) 8 containing measurement target gas 7, and a calculation section 9. The photoelectric sensor 4 has a housing 40 in which a light emission section 2, a light reception section 3, and a signal processing section 1 are integrally formed. The light emission section 2 emits visible light 10 as light.

In particular, a red light emitting diode with a four-element type made of AlInGAP (aluminum, indium, gallium, phosphide) is preferably used in terms of a wavelength, luminance, and stability, and allows measurement of an ozone concentration to be performed with high accuracy. The visible light 10 includes at least light having a wavelength of not less than 500 nm and not greater than 700 nm. This wavelength exhibits a large absorption cross section with ozone, thus allowing measurement of an ozone concentration to be performed with high accuracy.

The light reception section 3 receives the visible light 10. In the photoelectric sensor 4, the light emission section 2 and the light reception section 3 are arranged in parallel to the center axis of the container 8. The signal processing section 1 transmits a light emission signal for causing the light emission section 2 to emit the visible light 10 having a predetermined light emission intensity, and receives a light reception signal from the light reception section 3 that has received the visible light 10.

The light-emission optical fiber 5 is connected to the light emission section 2 and one end side of the container 8, and introduces the visible light 10 emitted from the light emission section 2, into the container 8 from the one end side of the container 8. The light-reception optical fiber 6 is connected to the other end side of the container 8 and the light reception section 3, receives the visible light 10 that has passed through the container 8, from the other end side of the container 8, and introduces the visible light 10 into the light reception section 3.

As the photoelectric sensor 4 formed as described above, a digital fiber sensor can be used, for example. As with the photoelectric sensor 4 according to the present invention, the digital fiber sensor includes the light emission section 2, the light reception section 3, and the signal processing section 1 inside the housing 40, and has a structure for connecting these components to optical fibers.

As in the present invention, the digital fiber sensor has a function of adjusting the light emission intensity of the light emission section 2 and a function of outputting a signal of a light reception intensity from the light reception section 3 to the outside, by the signal processing section 1. Therefore, the digital fiber sensor can be favorably applied to the ozone concentration measurement device 100 according to the present invention.

In addition, using the digital fiber sensor as the photoelectric sensor 4 makes it easy to obtain the photoelectric sensor 4 having high stability at low cost while reducing the device size, and thus is significantly effective for providing the ozone concentration measurement device 100 that can measure an ozone concentration with high accuracy, using a small-sized and simple configuration, as in the present invention.

As shown in FIG. 2, the container 8 is formed by a hollow pipe-shaped member, here, a straight pipe, and is formed by a stainless steel pipe, for example. The inner wall of the container 8 is treated through electropolishing or bright annealing, so as to have a high reflectance for the visible light 10. Thus, it is possible to measure an ozone concentration with high accuracy and with a simple configuration without using an optical component such as a lens or a collimator.

T-shaped connection pipes 11A and 11B are provided at both ends of the container 8. At the connection pipe 11A on one end side of the container 8, one end side of the container 8 is connected to a first socket portion 11A1, the light-emission optical fiber 5 is connected to a second socket portion 11A2 via a fiber connector 12A, and a third socket portion 11A3 is an inlet for the measurement target gas 7.

At the connection pipe 11B on the other end side of the container 8, the other end side of the container 8 is connected to a first socket portion 11B1, the light-reception optical fiber 6 is connected to a second socket portion 11B2 via a fiber connector 12B, and a third socket portion 11B3 is an outlet for the measurement target gas 7. For the connection pipes 11A and 11B, a material having high ozone resistance, e.g., stainless steel or fluororesin, is favorably used.

The fiber connectors 12A and 12B are made of a material that has ozone resistance and allows transmission of visible light. For example, glass or translucent fluororesin is favorably used. As described above, the light-emission optical fiber 5 and the light-reception optical fiber 6 are provided to the connection pipes 11A and 11B via the fiber connectors 12A and 12B, whereby the light-emission optical fiber 5 and the light-reception optical fiber 6 are prevented from being directly exposed to ozone in the measurement target gas 7 and deterioration of the light-emission optical fiber 5 and the light-reception optical fiber 6 is suppressed.

Thus, it becomes possible to perform measurement for an ozone concentration stably over a long period. In addition, the light-emission optical fiber 5 and the light-reception optical fiber 6 do not necessarily need to be made of a material having high chemical resistance, and therefore an inexpensive and general optical fiber can be used. Thus, the device cost can be reduced.

In addition, since a measurement section is composed of the container 8, the connection pipes 11A and 11B, and the fiber connectors 12A and 12B, a highly airtight structure can be easily obtained. Thus, it is possible to measure an ozone concentration stably even under a positive-pressure or negative-pressure condition. In addition, since the measurement section is composed of the container 8, the connection pipes 11A and 11B, and the fiber connectors 12A and 12B, the measurement section can be formed by only members having high thermal resistance. Thus, it is possible to measure an ozone concentration in the measurement target gas over a wide temperature range.

The calculation section 9 calculates an ozone concentration by Formula (1) described later, on the basis of information of the light reception signal (light reception intensity) sent from the photoelectric sensor 4. As the calculation section 9, a device having a function for performing such calculation may be used. For example, a PC (personal computer), a microcontroller, a PLC (programmable logic controller), an FPGA (field programmable gate array), or the like may be used.

Next, an ozone concentration measurement method using the ozone concentration measurement device 100 according to Embodiment 1 configured as described above will be described. First, an operation of introducing the measurement target gas 7 from the third socket portion 11A3 of the connection pipe 11A on one end side of the container 8 and leading out the measurement target gas 7 from the third socket portion 11B3 of the connection pipe 11B on the other end side of the container 8, is continuously performed to keep the inside of the container 8 filled with the measurement target gas 7.

Then, a photoelectric sensor preparation step is performed to prepare the photoelectric sensor 4 in which the light emission section 2 for emitting light, the light reception section 3 for receiving light, and the signal processing section 1 for transmitting a light emission signal to the light emission section 2 and receiving a light reception signal from the light reception section 3, are integrally formed.

Then, a light-emission optical fiber preparation step is performed to prepare the light-emission optical fiber 5 which is connected to the light emission section 2 and introduces light into the container 8. Then, a light-reception optical fiber preparation step is performed to prepare the light-reception optical fiber 6 which leads out light having passed through the inside of the container 8, to the light reception section 3.

Next, in the photoelectric sensor 4, the light emission section 2 emits the visible light 10 having a set light emission intensity on the basis of the light emission signal from the signal processing section 1. Thus, the visible light 10 passes through the light-emission optical fiber 5 and is emitted into the container 8 from one end side of the container 8 via the fiber connector 12A. The visible light 10 introduced into the container 8 propagates while being repeatedly reflected by the inner wall of the container 8, and reaches the light-reception optical fiber 6 via the fiber connector 12B on the other end side of the container 8.

Then, the light reception intensity is measured in the light reception section 3 and is transmitted to the signal processing section 1. Then, the calculation section 9 performs a calculation step of calculating an ozone concentration of the measurement target gas 7 on the basis of a signal of the light reception intensity from the signal processing section 1, as described below.

In a case where ozone is absent in the measurement target gas 7 in the container 8, the visible light 10 is not absorbed by the measurement target gas 7, and the light reception intensity of the visible light 10 measured by the light reception section 3 is not reduced. On the other hand, in a case where ozone is present in the measurement target gas 7 in the container 8, the visible light 10 is absorbed by ozone, so that the light reception intensity of the visible light 10 measured by the light reception section 3 is reduced. On the basis of the ratio of the light reception intensities sent from the photoelectric sensor 4, the calculation section 9 calculates an ozone concentration in the measurement target gas 7 in the container 8 by Formula (1) described below.

[Mathematical 1]

$$C_{O3} = \frac{-48 \times 10^3}{\epsilon L} log\left(\frac{I}{I_0}\right) \quad \cdots (1)$$

Here, $I_0$ is a light reception intensity when ozone is absent, $I$ is a light reception intensity when ozone is present, $\epsilon$ is an absorption coefficient ($dm^2/mol$) of ozone, $L$ is an optical path length (dm), and $C_{O3}$ is an ozone concentration ($g/m^3$). The absorption coefficient $\epsilon$ of ozone is a physical property value, and the optical path length $L$ is a constant determined by the size of the container 8. Therefore, the ozone concentration is calculated by the above Formula (1) from the light reception intensities when ozone is absent and when ozone is present.

Here, the example in which the measurement target gas 7 is introduced from the connection pipe 11A on the light-emission optical fiber 5 side and led out from the connection pipe 11B on the light-reception optical fiber 6 side, has been shown. However, the flow direction of the measurement target gas 7 is not limited. For example, the flow direction may be reversed so that the measurement target gas 7 is introduced from the connection pipe 11B on the light-reception optical fiber 6 side and led out from the connection pipe 11A on the light-emission optical fiber 5 side. Even in this case, the ozone concentration of the measurement target gas 7 can be measured in the same manner.

The optical path length of the container 8 is arbitrarily determined in accordance with the ozone concentration of the measurement target gas 7 to be measured, required measurement accuracy, and the like. However, it is preferable that the optical path length is not less than 10 cm and not more than 100 cm. The reason is as follows. If the optical path length is not more than 10 cm, light absorption by ozone does not sufficiently occur in the container 8, so that measurement accuracy for an ozone concentration might be reduced. If the optical path length is not less than 100 cm, when high-concentration ozone is present in the measurement target gas 7, it is difficult to perform measurement because light absorption by ozone is excessive, and in addition, the device size increases, so that the installation place is constrained.

In the present Embodiment 1, the example in which a straight pipe made of stainless steel is used as the container 8 has been shown. However, the container 8 is not limited thereto as long as the measurement target gas 7 can be contained therein and ozone concentration measurement using the visible light 10 can be performed. For example, the container 8 may have a metal-tank structure. Alternatively, the container 8 having high ozone resistance and high visible light reflection property may be obtained by forming a metal film on the outer periphery of a glass tube.

The ozone concentration measurement device according to Embodiment 1 configured as described above is an ozone concentration measurement device for measuring a concentration of ozone of measurement target gas in a container, the ozone concentration measurement device including: a photoelectric sensor in which a light emission section for emitting light, a light reception section for receiving the light, and a signal processing section for transmitting a light emission signal to the light emission section and receiving a light reception signal from the light reception section, are integrally formed; a light-emission optical fiber which is connected to the light emission section and introduces the light into the container; a light-reception optical fiber which leads out the light having passed through inside of the container, to the light reception section; and a calculation section which calculates the concentration of ozone of the measurement target gas on the basis of the light reception signal from the signal processing section.

Thus, since the light emission section and the light reception section of the photoelectric sensor are integrally formed, a temperature difference is less likely to arise between the light emission section and the light reception section, and reduction in measurement accuracy for an ozone concentration due to changes in the light emission intensity and the photosensitivity caused by the temperature difference can be suppressed.

Further, since the light emission section and the light reception section of the photoelectric sensor are integrally formed, the device configuration can be downsized and simplified.

Further, on the container side, only the light-emission optical fiber and the light-reception optical fiber are connected. Therefore, on the container side, electrical wiring is not needed and it is not necessary to install electrical wiring. Then, in the light emission section and the light reception section, it is possible to measure an ozone concentration stably with high accuracy without being influenced by temperature change in a wiring resistance and electromagnetic noise.

Further, since only the light-emission optical fiber and the light-reception optical fiber are connected on the container side, the container and the surrounding configurations are simplified and downsized, and thus the ozone concentration measurement device can be placed in a narrow space.

In the ozone concentration measurement device according to Embodiment 1 configured as described above, the container is formed by a hollow pipe-shaped member, the light-emission optical fiber is provided on one end side of the container, and the light-reception optical fiber is provided on another end side of the container.

Thus, the container can be formed with a simple configuration and light propagation is facilitated, so that measurement for an ozone concentration can be performed with high accuracy at low cost and using a simple configuration.

In the ozone concentration measurement device according to Embodiment 1 configured as described above, T-shaped connection pipes each having a first socket portion, a second socket portion, and a third socket portion are provided on the one end side and the other end side of the container. At the connection pipe on the one end side of the container, the one end side of the container is connected to the first socket portion, the light-emission optical fiber is connected to the second socket portion, and the third socket portion is an inlet for the measurement target gas. At the connection pipe on the other end side of the container, the other end side of the container is connected to the first socket portion, the light-reception optical fiber is connected to the second socket portion, and the third socket portion is an outlet for the measurement target gas.

Thus, with the connection pipes, provision of the optical fibers to the container and introduction and leading-out of the measurement target gas can be easily and simply performed, and the structure can be simplified.

Further, deterioration of the optical fibers can be suppressed, whereby measurement for an ozone concentration can be performed stably over a long period.

The ozone concentration measurement method according to Embodiment 1 configured as described above is an ozone concentration measurement method for measuring a concentration of ozone of measurement target gas in a container, the ozone concentration measurement method including: a photoelectric sensor preparation step of preparing a photoelectric sensor in which a light emission section for emitting light, a light reception section for receiving the light, and a signal processing section for transmitting a light emission signal to the light emission section and receiving a light reception signal from the light reception section, are integrally formed; a light-emission optical fiber preparation step of preparing a light-emission optical fiber which is connected to the light emission section and introduces the light into the container; a light-reception optical fiber preparation step of preparing a light-reception optical fiber which leads out the light having passed through inside of the container, to the light reception section; and a calculation step of calculating a concentration of ozone of the measurement target gas on the basis of the light reception signal from the signal processing section.

Thus, reduction in measurement accuracy for an ozone concentration due to changes in the light emission intensity and the photosensitivity caused by the temperature difference can be suppressed, and in addition, it is possible to measure an ozone concentration stably with high accuracy without being influenced by temperature change in a wiring resistance and electromagnetic noise.

Embodiment 2

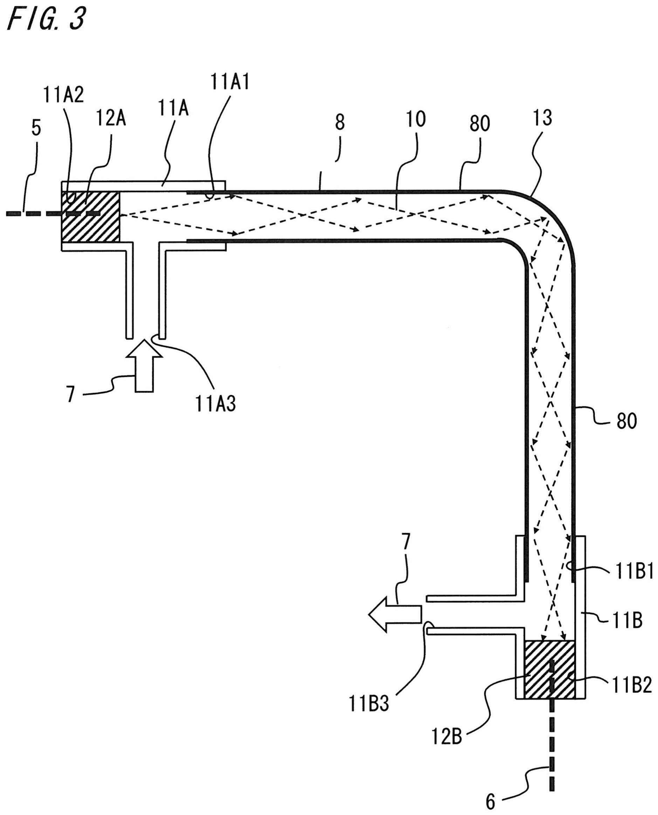

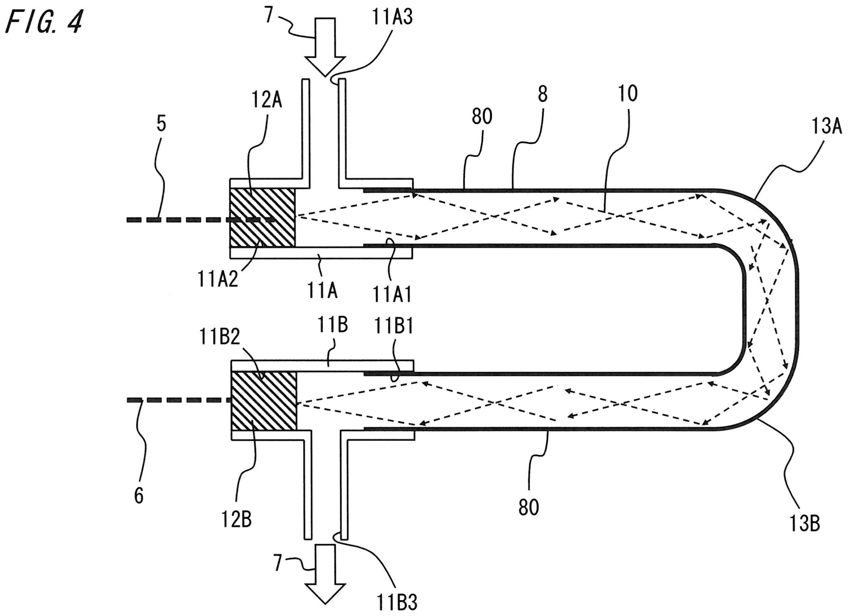

FIG. 3 and FIG. 4 are sectional views showing configurations of a container part of an ozone concentration measurement device according to Embodiment 2. As shown in FIG. 3, the present embodiment is different in that the container 8 has one bent pipe portion 13 in addition to a straight pipe portion 80, as compared to the above Embodiment 1. In another example shown in FIG. 4, the container 8 has two bent pipe portions 13A and 13B in addition to the straight pipe portion 80.

The inner wall of the container 8 is formed to have a high reflectance for the visible light 10, and thus the visible light 10 emitted from the light-emission optical fiber 5 propagates toward the light-reception optical fiber 6 while being reflected a plurality of times by the inner wall of the container 8. Therefore, even in the case where the bent pipe portions 13, 13A, 13B are present other than the straight pipe portion 80 as shown in FIG. 3 or FIG. 4, the visible light 10 can propagate while being repeatedly reflected, whereby an ozone concentration can be measured in the same manner as in the above Embodiment 1.

By providing the bent pipe portions 13, 13A, 13B to the container 8 as described above, the degree of freedom in the shape of the ozone concentration measurement device 100 can be improved. For example, the bent pipe portions 13, 13A, 13B may be formed as appropriate along a pipe of a provided ozone generator, whereby the degree of freedom in installation can be enhanced. In addition, providing the bent pipe portions 13, 13A, 13B makes it possible to form the container 8 having a great optical path length in a small size, and thus it becomes possible to perform high-accuracy ozone concentration measurement even with the ozone concentration measurement device 100 having a comparatively small size.

The bent pipe portions 13, 13A, 13B shown in FIG. 3 and FIG. 4 have a right angle, but the bending angle does not necessarily need to be a right angle and can be arbitrarily determined. For example, a pipe portion bent annularly or helically may be used, and even in this case, the same effects can be obtained.

The ozone concentration measurement device according to Embodiment 2 configured as described above provides the same effects as in the above Embodiment 1, and in addition,

the container is formed by a straight pipe portion and a bent pipe portion, the light-emission optical fiber is provided on the one end side of the container, and the light-reception optical fiber is provided on the other end side of the container.

Thus, the degree of freedom in the shape of the ozone concentration measurement device can be improved.

Embodiment 3

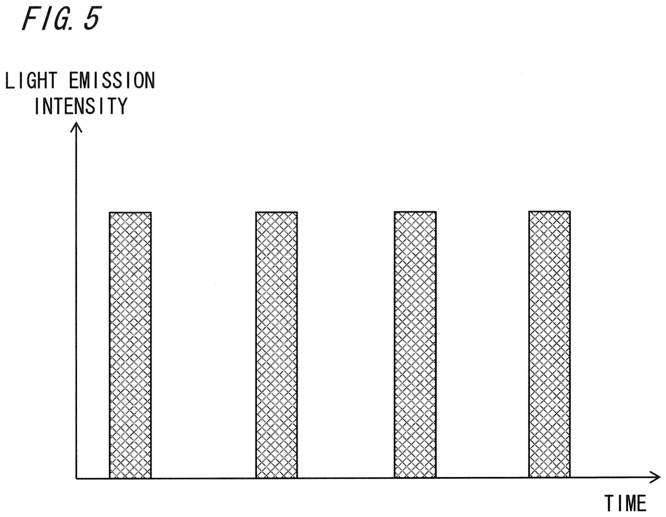

FIG. 5 shows a light emission pattern in the light emission section 2 of the photoelectric sensor 4 of an ozone concentration measurement device according to Embodiment 3.

In the present embodiment, a light emission pattern of the visible light 10 from the light emission section 2 of the photoelectric sensor 4 is subjected to pulse modulation. The other configurations and operations are the same as in the above embodiments. When the light emission section 2 continuously emits light, the temperature may increase. In general, the intensity and the wavelength of light emitted from the light emission section 2 (light emission element) are temperature-dependent. Therefore, while an ozone concentration is continuously measured, if the temperature of the light emission section 2 increases, measurement accuracy might be reduced. In the present embodiment, light emission of the light emission section 2 is subjected to pulse modulation so that light is intermittently emitted. Thus, as compared to a case of continuous light emission, heat generation is suppressed, whereby reduction in measurement accuracy for an ozone concentration due to temperature increase can be suppressed.

Embodiment 4

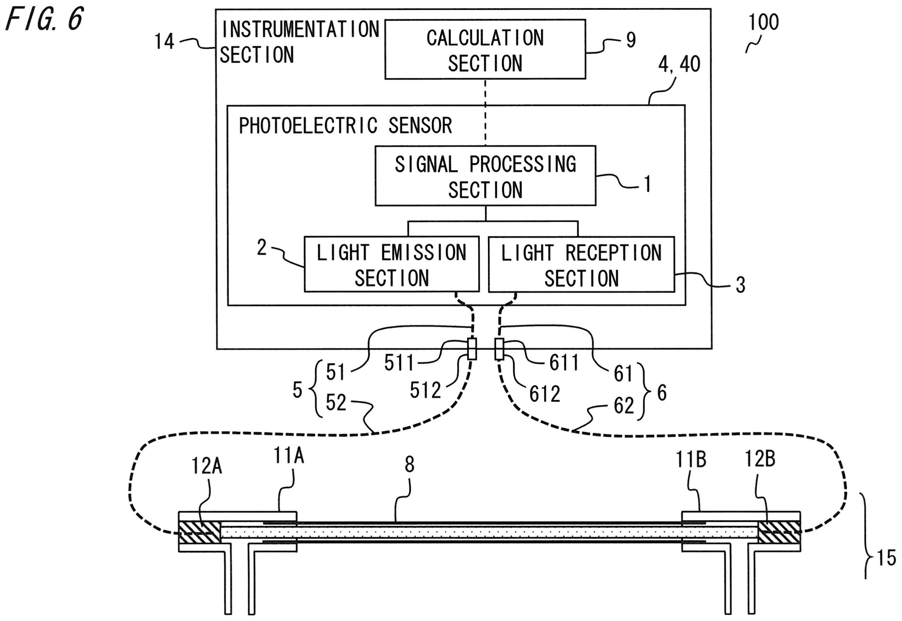

FIG. 6 shows a configuration of an ozone concentration measurement device according to Embodiment 4. In the present embodiment, the photoelectric sensor 4 and the calculation section 9 of the ozone concentration measurement device 100 are formed as an instrumentation section 14, and the container 8 side is defined as a measurement section 15. Thus, separate sections are formed unlike the above embodiments. For this configuration, the light-emission optical fiber 5 and the light-reception optical fiber 6 are composed of a plurality of optical fibers 51 and 52 and a plurality of optical fibers 61 and 62, respectively. Then, connection portions 511 and 512 and connection portions 611 and 612 for connecting the above optical fibers are provided. Here, the connection portions 511 and 611 are provided at the instrumentation section 14.

In this configuration, the instrumentation section 14 and the measurement section 15 are connected by being coupled via the connection portions 511, 512, 611, and 612 of the light-emission optical fiber 5 and the light-reception optical fiber 6, and thus these sections are independent of each other in terms of structure. Therefore, the instrumentation section 14 and the measurement section 15 can be provided separately from each other. As a result, even in a narrow place with a limited installation space, the ozone concentration measurement device 100 can be installed and can perform measurement for an ozone concentration. In the measurement section 15, no electric devices are included, and the measurement section 15 and the instrumentation section 14 are connected by only the optical fibers 5 and 6.

Therefore, for example, it becomes possible that only the instrumentation section 14 is provided at a place that is in a low-electromagnetic-noise environment and is indoor so as to be protected from wind and rain, and the measurement section 15 is provided at an outdoor place for measurement, for example. In addition, it becomes possible to provide the instrumentation section 14 at the same place as a control section of another device such as an ozone generation system, for example.

Here, the example in which each of the light-emission optical fiber 5 and the light-reception optical fiber 6 is composed of two optical fibers 51, 52, 61, 62 has been shown. However, without limitation thereto, three or more optical fibers are used and connection portions may be provided to the respective optical fibers.

Since the connection portions 511 and 611 are provided at the instrumentation section 14, attachment and detachment of the connection portions 511, 512, 611, and 612 can be easily performed and change in the light intensity due to movement or bending of the optical fibers 51, 52, 61, and 62 can be suppressed.

The ozone concentration measurement device according to Embodiment 4 configured as described above provides the same effects as in the above embodiments, and in addition,
each of the light-emission optical fiber and the light-reception optical fiber is composed of a plurality of optical fibers and is provided with connection portions connecting the plurality of optical fibers.

Thus, since the side where the calculation section and the photoelectric sensor are provided and the container side can be formed separately from each other in terms of structure via the optical fibers, it becomes possible to provide only the calculation section and the photoelectric sensor at a place that is in a low-electromagnetic-noise environment and is indoor so as to be protected from wind and rain. In addition, it becomes possible to configure the calculation section together with a control section of another device.

Embodiment 5

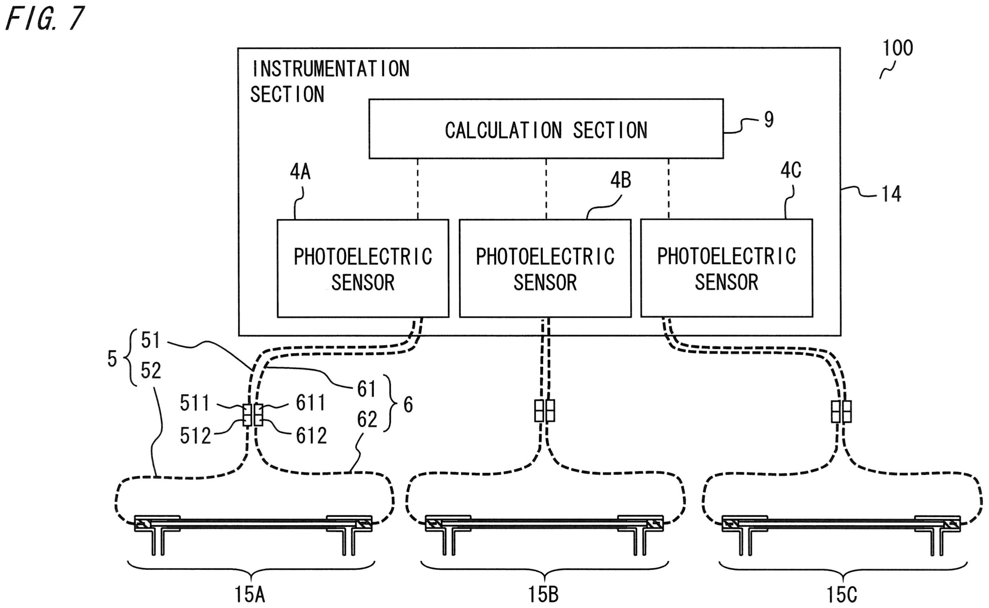

FIG. 7 shows a configuration of an ozone concentration measurement device according to Embodiment 5. In the present embodiment, the instrumentation section 14 includes a plurality of photoelectric sensors 4A, 4B, and 4C, the photoelectric sensors 4A, 4B, and 4C respectively correspond to measurement sections 15A, 15B, and 15C which are independent of each other, and the respective pairs are connected via the optical fibers 5 and 6. The other configurations are the same as in the above Embodiment 4.

In the present embodiment, in a case of measuring ozone concentrations at a plurality of locations, or in a case of measuring concentrations of ozone generated from a plurality of ozone generators, separate measurement sections 15A, 15B, and 15C are provided, the respective photoelectric sensors 4A, 4B, and 4C perform measurement, and the measurements can be aggregated in the instrumentation section 14. Thus, the ozone generation system can be simply configured. In addition, calculation sections can be unified as one calculation section 9. Therefore, the configuration cost can be reduced as compared to a case of providing independent calculation sections 9 for the plurality of measurement sections 15.

The ozone concentration measurement device according to Embodiment 5 configured as described above provides the same effects as in the above embodiments, and in addition,
a plurality of the photoelectric sensors are provided, the light-emission optical fiber, the light-reception optical fiber, and the container are provided for each of the photoelectric sensors, and the calculation section calculates the concentration of ozone of the measurement target gas for each of the containers connected to the respective photoelectric sensors.

Thus, the ozone concentration measurement device can be configured simply and at low cost, in a case of measuring ozone concentrations at a plurality of locations and in a case of measuring concentrations of ozone generated from a plurality of ozone generators.

Embodiment 6

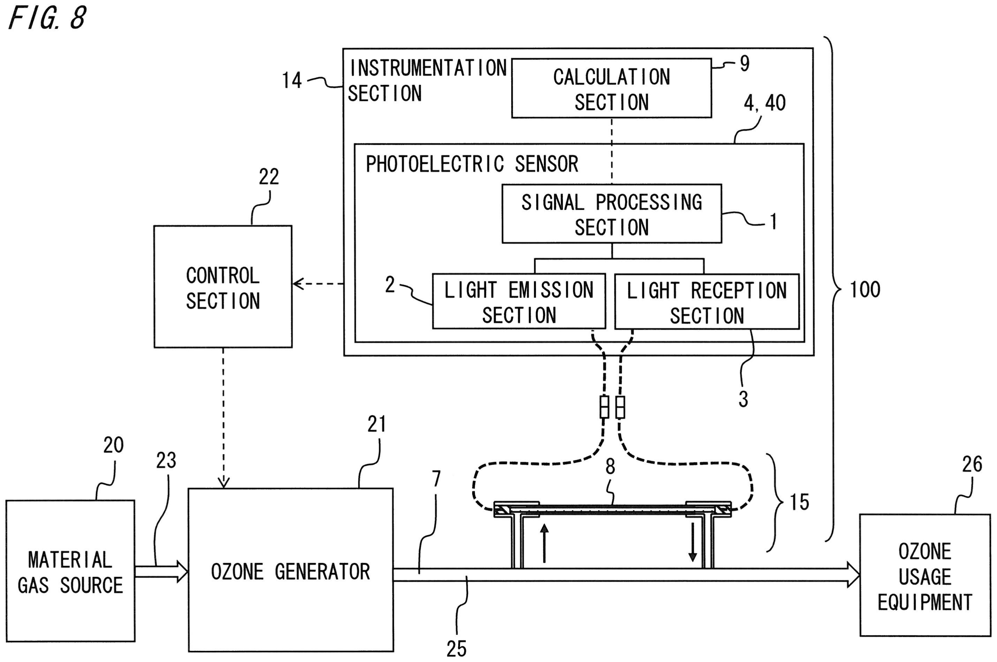

FIG. 8 shows a configuration of an ozone generation system according to Embodiment 6. As shown in FIG. 8, in the ozone generation system, material gas 23 containing oxygen is supplied from a material gas source 20 to an ozone generator 21. Then, ozone generated by the ozone generator 21 passes through an ozone pipe 25 and is supplied to ozone usage equipment 26, as measurement target gas 7. Then, the ozone generation system includes a control section 22 for controlling the ozone generator 21.

In the ozone concentration measurement device 100, ozone flowing through the ozone pipe 25 is split as measurement target gas 7, the measurement target gas 7 is taken into the container 8, and the ozone concentration thereof is measured. Then, the measurement target gas 7 is returned to the ozone pipe 25. The ozone concentration measurement device 100 does not necessarily need to be provided in a branched manner from the ozone pipe 25 as shown in FIG. 8, and the container 8 itself can be used as the ozone pipe 25, whereby the same operation can be performed.

The control section 22 performs feedback control of the operation condition of the ozone generator 21 so that ozone having a predetermined concentration can be stably supplied, on the basis of information on the ozone concentration measured by the ozone concentration measurement device 100. Examples of the ozone generation condition to be controlled include power supplied to the ozone generator 21, the flow rate of the material gas 23, and the composition of the material gas 23. However, without limitation thereto, any condition relevant to operation of the ozone generator 21 may be used.

The ozone generation system can measure the concentration of ozone generated by the ozone generator 21, in line and in real time, by the ozone concentration measurement device 100 capable of measuring the ozone concentration with high accuracy using a small-sized and simple configuration. In addition, feedback control is performed for the ozone generator 21 on the basis of information on the measured ozone concentration, whereby ozone having a predetermined concentration can be stably supplied to the ozone usage equipment 26.

The ozone generation system according to Embodiment 6 configured as described above includes: an ozone generator which generates and feeds ozone as the measurement target gas to outside; the above ozone concentration measurement device which introduces the measurement target gas fed from the ozone generator, into the container, and measures the concentration of ozone of the measurement target gas; and a control section which controls the ozone generator on the basis of a result of measurement for the concentration of ozone of the measurement target gas by the ozone concentration measurement device.

Thus, since the ozone concentration can be measured with a simple configuration, the ozone generator can be controlled so as to achieve a predetermined ozone concentration in accordance with the measured ozone concentration, and the ozone generation system can be simply configured.

Embodiment 7

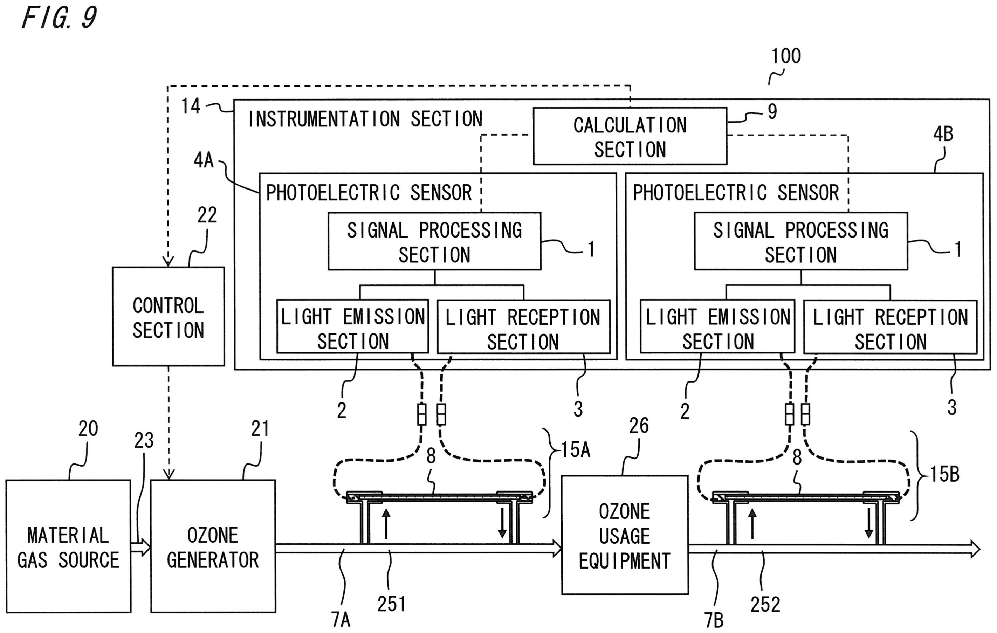

FIG. 9 shows a configuration of an ozone generation system according to Embodiment 7. In the present embodiment, measurement target gas passing through an ozone pipe 251 on the upstream side and introduced into the ozone usage equipment 26 is first measurement target gas 7A, and a measurement section 15A for measuring an ozone concentration of the first measurement target gas 7A is provided. In addition, measurement target gas discharged from the ozone usage equipment 26 and passing through an ozone pipe 252 on the downstream side is second measurement target gas 7B, and a measurement section 15B for measuring an ozone concentration of the second measurement target gas 7B is provided. The measurement section 15A and the measurement section 15B are connected to the instrumentation section 14 via the optical fibers 5 and 6. The ozone concentration measurement device 100 is composed of the measurement section 15A, the measurement section 15B, and the instrumentation section 14.

The ozone concentration measurement device 100 measures both of the ozone concentration in the measurement section 15A, i.e., the ozone concentration of the first measurement target gas 7A on the upstream side of the ozone usage equipment 26, and the ozone concentration in the measurement section 15B, i.e., the ozone concentration of the second measurement target gas on the downstream side of the ozone usage equipment 26, and sends resultant signals to the control section 22. The control section 22 performs feedback control of the operation condition of the ozone generator 21 in accordance with the sent information on the ozone concentrations. The other configurations and operations are the same as in the above Embodiment 6.

The ozone usage equipment 26 is, for example, a water treatment tank. In the present embodiment, the ozone concentrations on the upstream side and the downstream side of the water treatment tank are measured at the same time, to calculate an ozone consumption amount, whereby it becomes possible to perform high-level control such as minimizing the amount of ozone to leak to the downstream side. In addition, one instrumentation section 14 can be shared between two measurement sections 15A and 15B, whereby it is possible to measure ozone concentrations at two locations with a small-sized and simple configuration.

The ozone generation system according to Embodiment 7 configured as described above includes: an ozone generator which generates ozone and feeds first measurement target gas as the measurement target gas to outside; ozone usage equipment which receives the first measurement target gas from the ozone generator, uses ozone thereof, and discharges second measurement target gas as the measurement target gas; the above ozone concentration measurement device, which introduces, into the container, the first measurement target gas to be introduced into the ozone usage equipment, and measures the concentration of ozone of the first measurement target gas, and which introduces, into another said container different from the container, the second measurement target gas discharged from the ozone usage equipment, and measures the concentration of ozone of the second measurement target gas; and a control section which controls the ozone generator on the basis of a result of measurement for the concentrations of ozone of the first measurement target gas and the second measurement target gas by the ozone concentration measurement device.

Thus, since ozone concentrations can be measured with simple configurations before and after the ozone usage equipment, it is possible to configure an ozone generation system that can perform control so as to optimize the amount of ozone generation by the ozone generator in accordance with an ozone usage amount.

It is possible to configure an ozone generation system that can generate measurement target gas (ozone gas) having a predetermine ozone concentration, with a simple configuration.

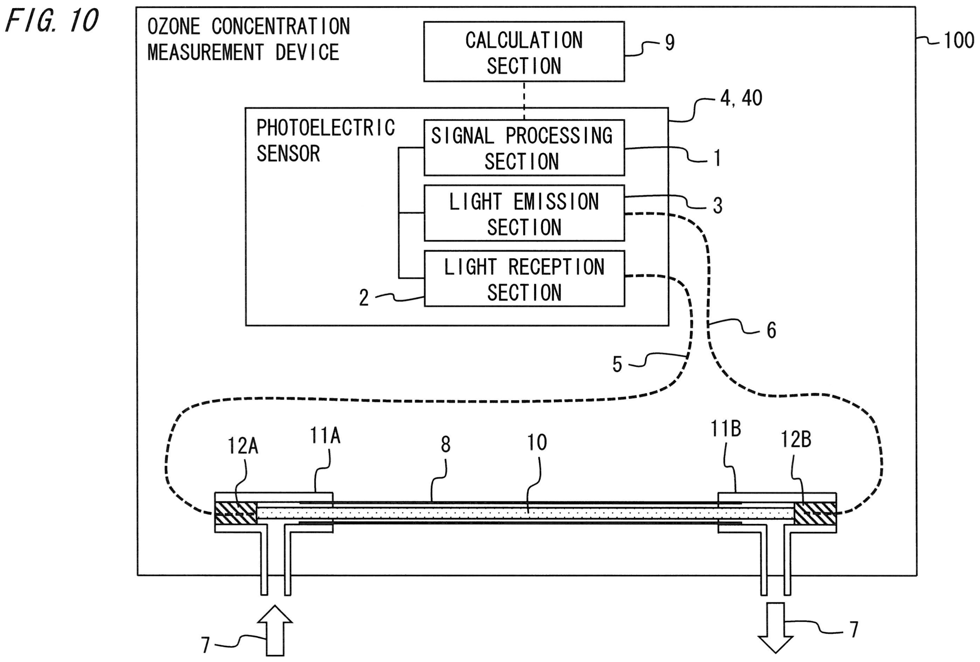

In the above embodiments, the example in which the light emission section 2 and the light reception section 3 of the photoelectric sensor 4 are arranged in parallel to the center axis of the container 8 has been shown. However, without limitation thereto, as shown in FIG. 10, the light emission section 2 and the light reception section 3 may be arranged perpendicularly to the center axis of the container 8. Thus, the photoelectric sensor 4 can be formed in a small size, so that flexibility in installation is improved.

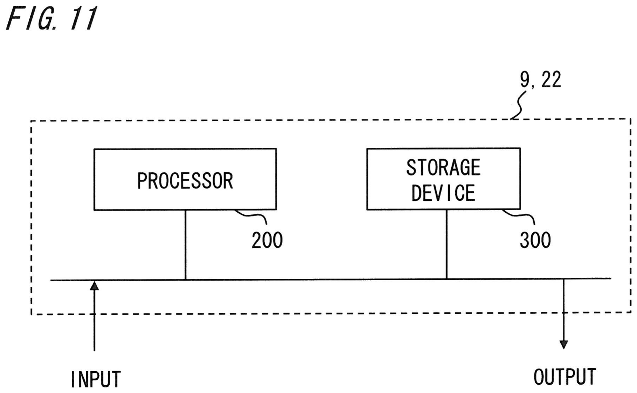

As shown in a hardware example in FIG. 11, the calculation section 9 and the control section 22 are composed of a processor 200 and a storage device 300. Although not shown, the storage device is provided with a volatile storage device such as a random access memory and a nonvolatile auxiliary storage device such as a flash memory. Instead of the flash memory, an auxiliary storage device of a hard disk may be provided. The processor 200 executes a program inputted from the storage device 300. In this case, the program is inputted from the auxiliary storage device to the processor 200 via the volatile storage device. The processor 200 may output data such as a calculation result to the volatile storage device of the storage device 300, or may store such data into the auxiliary storage device via the volatile storage device.

Although the invention is described above in terms of various exemplary embodiments and implementations, it should be understood that the various features, aspects, and functionality described in one or more of the individual embodiments are not limited in their applicability to the particular embodiment with which they are described, but instead can be applied, alone or in various combinations to one or more of the embodiments of the invention.

It is therefore understood that numerous modifications which have not been exemplified can be devised without departing from the scope of the present invention. For example, at least one of the constituent components may be modified, added, or eliminated. At least one of the constituent components mentioned in at least one of the preferred embodiments may be selected and combined with the constituent components mentioned in another preferred embodiment.

**LIST OF REFERENCE SIGNS**

1 signal processing section
10 visible light
100 ozone concentration measurement device
11A connection pipe
11B connection pipe
12A fiber connector
12B fiber connector
13 bent pipe portion
13A bent pipe portion
13B bent pipe portion
14 instrumentation section
15 measurement section
15A measurement section
15B measurement section
15C measurement section
2 light emission section
20 material gas source
21 ozone generator
22 control section
23 material gas
25 ozone pipe

251 ozone pipe
252 ozone pipe
26 ozone usage equipment
3 light reception section
4 photoelectric sensor
4A photoelectric sensor
4B photoelectric sensor
4C photoelectric sensor
40 housing
5 light-emission optical fiber
51 optical fiber
511 connection portion
512 connection portion
52 optical fiber
6 light-reception optical fiber
61 optical fiber
611 connection portion
612 connection portion
62 optical fiber
7 measurement target gas
7A first measurement target gas
7B second measurement target gas
8 container
80 straight pipe portion
9 calculation section

**Claims**

1. An ozone concentration measurement device for measuring a concentration of ozone of measurement target gas in a container, the ozone concentration measurement device comprising:

   a photoelectric sensor in which a light emission section for emitting light, a light reception section for receiving the light, and a signal processing section for transmitting a light emission signal to the light emission section and receiving a light reception signal from the light reception section, are integrally formed;
   a light-emission optical fiber which is connected to the light emission section and introduces the light into the container;
   a light-reception optical fiber which leads out the light having passed through inside of the container, to the light reception section; and
   a calculation section which calculates the concentration of ozone of the measurement target gas on the basis of the light reception signal from the signal processing section.

2. The ozone concentration measurement device according to claim 1, wherein

   the container is formed by a hollow pipe-shaped member,
   the light-emission optical fiber is provided on one end side of the container,
   the light-reception optical fiber is provided on another end side of the container.

3. The ozone concentration measurement device according to claim 2, wherein

   the container is formed by a straight pipe portion and a bent pipe portion,
   the light-emission optical fiber is provided on the one end side of the container, and
   the light-reception optical fiber is provided on the other end side of the container.

4. The ozone concentration measurement device according to any one of claims 1 to 3, wherein
   each of the light-emission optical fiber and the light-reception optical fiber is composed of a plurality of optical fibers and is provided with connection portions connecting the plurality of optical fibers.

5. The ozone concentration measurement device according to any one of claims 1 to 4, wherein
the light emission section and the light reception section in the photoelectric sensor are arranged in parallel to a center axis of the container, or arranged perpendicularly to the center axis of the container.

6. The ozone concentration measurement device according to any one of claims 1 to 5, wherein

T-shaped connection pipes each having a first socket portion, a second socket portion, and a third socket portion are provided on the one end side and the other end side of the container,
at the connection pipe on the one end side of the container, the one end side of the container is connected to the first socket portion, the light-emission optical fiber is connected to the second socket portion, and the third socket portion is an inlet for the measurement target gas, and
at the connection pipe on the other end side of the container, the other end side of the container is connected to the first socket portion, the light-reception optical fiber is connected to the second socket portion, and the third socket portion is an outlet for the measurement target gas.

7. The ozone concentration measurement device according to any one of claims 1 to 6, wherein

a plurality of the photoelectric sensors are provided,
the light-emission optical fiber, the light-reception optical fiber, and the container are provided for each of the photoelectric sensors, and
the calculation section calculates the concentration of ozone of the measurement target gas for each of the containers connected to the respective photoelectric sensors.

8. An ozone generation system comprising:

an ozone generator which generates and feeds ozone as the measurement target gas to outside;
the ozone concentration measurement device according any one of claims 1 to 7, which introduces the measurement target gas fed from the ozone generator, into the container, and measures the concentration of ozone of the measurement target gas; and
a control section which controls the ozone generator on the basis of a result of measurement for the concentration of ozone of the measurement target gas by the ozone concentration measurement device.

9. An ozone generation system comprising:

an ozone generator which generates ozone and feeds first measurement target gas as the measurement target gas to outside;
ozone usage equipment which receives the first measurement target gas from the ozone generator, uses ozone thereof, and discharges second measurement target gas as the measurement target gas;
the ozone concentration measurement device according to claim 7, which introduces, into the container, the first measurement target gas to be introduced into the ozone usage equipment, and measures the concentration of ozone of the first measurement target gas, and which introduces, into another said container different from the container, the second measurement target gas discharged from the ozone usage equipment, and measures the concentration of ozone of the second measurement target gas; and
a control section which controls the ozone generator on the basis of a result of measurement for the concentrations of ozone of the first measurement target gas and the second measurement target gas by the ozone concentration measurement device.

10. An ozone concentration measurement method for measuring a concentration of ozone of measurement target gas in a container, the ozone concentration measurement method comprising:

a photoelectric sensor preparation step of preparing a photoelectric sensor in which a light emission section for emitting light, a light reception section for receiving the light, and a signal processing section for transmitting a light emission signal to the light emission section and receiving a light reception signal from the light reception section, are integrally formed;
a light-emission optical fiber preparation step of preparing a light-emission optical fiber which is connected to the light emission section and introduces the light into the container;
a light-reception optical fiber preparation step of preparing a light-reception optical fiber which leads out the light having passed through inside of the container, to the light reception section; and

a calculation step of calculating a concentration of ozone of the measurement target gas on the basis of the light reception signal from the signal processing section.

EP 4 745 558 A1

**FIG. 1**

OZONE CONCENTRATION MEASUREMENT DEVICE ~100

CALCULATION SECTION ~9

4, 40

PHOTOELECTRIC SENSOR

SIGNAL PROCESSING SECTION ~1

2 ~ LIGHT EMISSION SECTION

LIGHT RECEPTION SECTION ~3

5

6

12A 11A 8 10 11B 12B

7 7

FIG. 2

*FIG. 3*

FIG. 4

*FIG. 5*

LIGHT EMISSION INTENSITY

TIME

FIG. 6

FIG. 7

FIG. 8

INSTRUMENTATION SECTION 14

CALCULATION SECTION 9

PHOTOELECTRIC SENSOR 4, 40

SIGNAL PROCESSING SECTION 1

LIGHT EMISSION SECTION 2

LIGHT RECEPTION SECTION 3

CONTROL SECTION 22

100

MATERIAL GAS SOURCE 20

OZONE GENERATOR 21

OZONE USAGE EQUIPMENT 26

23

7

8

15

25

EP 4 745 558 A1

FIG. 9

FIG. 10

EP 4 745 558 A1

FIG. 11

9, 22

| PROCESSOR | STORAGE DEVICE |
|---|---|

200

300

INPUT

OUTPUT

## INTERNATIONAL SEARCH REPORT

| International application No. |
| --- |
| **PCT/JP2023/030506** |

| A. | CLASSIFICATION OF SUBJECT MATTER |
| --- | --- |

*G01N 21/27*(2006.01)i
FI:  G01N21/27 Z

According to International Patent Classification (IPC) or to both national classification and IPC

| B. | FIELDS SEARCHED |
| --- | --- |

Minimum documentation searched (classification system followed by classification symbols)

G01N21/00-21/01; G01N21/17-21/61

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | JP 2014-115200 A (MITSUBISHI HEAVY INDUSTRIES, LTD.) 26 June 2014 (2014-06-26) paragraphs [0017]-[0037], fig. 1-7 | 1-10 |
| Y | JP 52-130682 A (MITSUBISHI ELECTRIC CORPORATION) 02 November 1977 (1977-11-02) publication gazette, p. 1, right column, line 4 to p. 2, lower left column, line 8, fig. 1 | 1-10 |
| Y | WO 2020/158506 A1 (FUJIKIN INC.) 06 August 2020 (2020-08-06) paragraphs [0018]-[0019], [0029]-[0037], fig. 1 | 1-10 |
| Y | WO 2017/199721 A1 (KYOTO INSTITUTE OF TECHNOLOGY) 23 November 2017 (2017-11-23) paragraphs [0019]-[0026], fig. 1 | 1-10 |
| Y | JP 3-215730 A (SUMITOMO ELECTRIC INDUSTRIES, LTD.) 20 September 1991 (1991-09-20) publication gazette, p. 2, lower right column, line 20 to p. 3, upper left column, line 7, p. 3, lower left column, lines 7-11, fig. 1 | 4-9 |

☑ Further documents are listed in the continuation of Box C.   ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **11 October 2023** | **24 October 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| International application No. |
| --- |
| **PCT/JP2023/030506** |

### C. DOCUMENTS CONSIDERED TO BE RELEVANT

| Category* | Citation of document, with indication, where appropriate, of the relevant passages | Relevant to claim No. |
| --- | --- | --- |
| Y | JP 2015-175773 A (MITSUBISHI ELECTRIC CORPORATION) 05 October 2015 (2015-10-05)<br>paragraph [0010], fig. 1 | 7-9 |
| Y | JP 2006-198544 A (KABUSHIKI KAISHA TOSHIBA) 03 August 2006 (2006-08-03)<br>paragraphs [0036]-[0038], fig. 1 | 8 |
| Y | JP 2002-263480 A (KANSAI ELECTRIC POWER CO., INC.) 17 September 2002 (2002-09-17)<br>paragraphs [0025]-[0026], fig. 2 | 9 |
| A | US 2005/0025965 A1 (SANGHERA, et al.) 03 February 2005 (2005-02-03) | 1-10 |
| A | US 2015/0102240 A1 (BEIJING INFORMATION SCIENCE & TECHNOLOGY UNIVERSITY) 16 April 2015 (2015-04-16) | 1-10 |

Form PCT/ISA/210 (second sheet) (January 2015)

## INTERNATIONAL SEARCH REPORT
### Information on patent family members

International application No.

**PCT/JP2023/030506**

| Patent document cited in search report | | | Publication date (day/month/year) | Patent family member(s) | | | Publication date (day/month/year) |
|---|---|---|---|---|---|---|---|
| JP | 2014-115200 | A | 26 June 2014 | (Family: none) | | | |
| JP | 52-130682 | A | 02 November 1977 | (Family: none) | | | |
| WO | 2020/158506 | A1 | 06 August 2020 | US | 2022/0074851 | A1 | |
| | | | | paragraphs [0026]-[0027], [0037]-[0045], fig. 1 | | | |
| | | | | KR | 10-2021-0052550 | A | |
| | | | | CN | 113260850 | A | |
| | | | | TW | 202041844 | A | |
| WO | 2017/199721 | A1 | 23 November 2017 | (Family: none) | | | |
| JP | 3-215730 | A | 20 September 1991 | (Family: none) | | | |
| JP | 2015-175773 | A | 05 October 2015 | (Family: none) | | | |
| JP | 2006-198544 | A | 03 August 2006 | CN | 101164914 | A | |
| JP | 2002-263480 | A | 17 September 2002 | (Family: none) | | | |
| US | 2005/0025965 | A1 | 03 February 2005 | US | 2005/0074215 | A1 | |
| | | | | US | 2006/0230792 | A1 | |
| | | | | US | 2007/0110377 | A1 | |
| | | | | US | 2008/0060387 | A1 | |
| | | | | US | 2010/0202743 | A1 | |
| | | | | WO | 2005/017569 | A2 | |
| | | | | WO | 2006/047316 | A2 | |
| | | | | CA | 2534696 | A1 | |
| US | 2015/0102240 | A1 | 16 April 2015 | CN | 103487402 | A | |

Form PCT/ISA/210 (patent family annex) (January 2015)

**EP 4 745 558 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012132827 A